# EUROPEAN PATENT APPLICATION

(11) **EP 4 417 683 A1**
(43) Date of publication of application: **21.08.2024**
(21) Application number: 22879615.7
(22) Date of filing: 07.10.2022
(51) Int. Cl.: C12M 3/06, C12M 1/00, C12M 3/00

(54) **MICROFLUIDIC DEVICE FOR BIOLOGICAL TISSUE STIMULATION FACILITATING CONTROL OF FLUID FLOW INSIDE CHANNEL**

(30) Priority: 15.10.2021 KR 20210137294
(71) Applicant: Edmicbio Inc., Seoul 02455 (KR)
(72) Inventor: HA, Dong Heon, Seoul 02455 (KR)
(74) Representative: Schnappauf, Georg
(86) International application number: PCT/KR2022/015164
(87) International publication number: WO 2023/063660

(57) **Abstract**

The present disclosure relates to a microfluidic device for mimicking biological tissue capable of easily controlling a fluid flow in a channel and a method of culturing a cell using the same, more specifically, a microfluidic device for mimicking biological tissue and a method of culturing a cell using the same through a tilt, which not only can make a fluid flow inside the channel be easily controlled, but also can culture and observe cells, without separating cells from each other, by independently supplying fluids containing necessary nutrients to the cells on the outer side and inner side of the tissue through the tilt of the device, without requiring a separate pump.

## Description

### CROSS REFERENCE TO RELATED APPLICATION

The present application claims priority to Korea Patent Application No. 10-2021-0137294, filed on October 15, 2021, the entire contents of which is incorporated herein for all purposes by this reference.

### Field

The present disclosure relates to a microfluidic device for mimicking biological tissue capable of easily controlling a fluid flow in a channel and a method of culturing a cell using the same, more specifically, a microfluidic device for mimicking biological tissue and a method of culturing a cell using the same through a tilt, which not only can make a fluid flow inside the channel be easily controlled, but also can culture and observe cells, without separating cells from each other, by independently supplying fluid containing necessary nutrients to the cells on an outer side and an inner side of the tissue through the tilt of the device, without requiring a separate pump. The present disclosure was supported by Business for Startup growth and technological development (TIPS Program) funded Korea Ministry of SMEs and Startups in 2020 (Grants No. S3032532).

### Background

The most actively researched field at the interface between IT and BT technology is a biochip field. Biochips may be largely classified into microarray chips and microfluidic chips. In this field, the microfluidic chip technology is a technology that measures and analyzes an interaction of a material to be analyzed contained in a fluid sample with a biological material, cell, tissue, or detection device on a chip using microfluidic control technology. Related prior literature is introduced below. Reference numerals in the following prior documents are irrelevant to the present disclosure.

Korean Patent No. 10-1898093, 'Analysis Chip Containing Biological Tubule Mimetic Tissue and Method of Manufacturing the Same' relates to a method of manufacturing a chip for analyzing an analysis chip containing biological tubule mimetic tissue and the chip containing biological tubule mimetic tissue. Regarding the analysis chip containing the biological tubule mimetic tissue of the present disclosure, the biological tubule formed of the epithelial cell membrane and the extracellular matrix surrounding the biological tubule were implemented as closely as possible to the biological environment on the chip for analysis. Therefore, the analysis chip containing the biological tubule mimetic tissue of the present disclosure may provide a platform through which research on the process of carcinogenesis or metastasis of cancer cells or screening or testing of new anticancer drugs can be conducted similar to actual in vivo conditions.

Korean Patent No. 20220012976A, 'a microfluidic system simulating a lung tissue' relates to a microfluidic system simulating a lung tissue which includes lung epithelial cells and lung fibroblasts which are isolated from human lungs and commercially available vascular endothelial cells, and in which microfluid is perfused, a method for manufacturing the same, and a microfluidic control method using the same. Each chamber inside the corresponding system can allow a fluid, which contains gas and a medium, to flow therethrough and simulate respiration-like movement, wherein all of the three types of cells can survive inside the system even when one week or more have elapsed after through-flow of the fluid. In addition, the pH and pO₂ in the chamber can be monitored by using a pH sensor and a gas partial pressure sensor inside the system, and thus the three types of cells inside the system can be exposed to external environments, drugs, and the like under the same conditions as in the lungs in vivo. Therefore, a wide range of studies including modeling of lung diseases by harmful substances and testing of therapeutic drug efficacy can be conducted, and further, the utilization to in vitro disease modeling, customized medicine prescriptions, and the like can also be made.

However, the technology of the prior literature had a limit to mimicking biological tissue since the tissue is cultured in a state in which cells present in the outer and inner sides of the tissue are separated.

### SUMMARY

The technical problem to be achieved by the present disclosure is providing a microfluidic device for mimicking biological tissue and a method of culturing cells using the same, which is capable of independently supplying nutrients optimized to the cells without separating the cells present in the outer and inner sides of the tissue, by not only easily controlling a fluid flow inside the channel through a tilt without requiring a separate pump, but also facilitating the flow of different fluids supplied to the outer and inner sides of the tissue matrix through the tilt.

The technical problem to be achieved by the present disclosure is not limited to the above-mentioned technical problem, and other technical problems that are not mentioned will be clearly understood by ordinary-skilled persons in the art to which the present disclosure pertains from the following description.

One embodiment is a microfluidic device for mimicking biological tissue, including: a frame 100; and a conduit installation part 200 for holding a biomimetic conduit 210 installed inside the frame 100, and the microfluidic device includes a first channel 300 having at least one partition wall 310 partitioning an internal passage at a certain height in a transverse direction and having one side allowing a tilted surface formed therein such that a fluid flow inside the first channel 300 is easily controllable through a tilt.

The microfluidic device may further include: a second channel 400 arranged in parallel with the first channel, and the second channel 400 may have at least one or more partition walls 410 partitioning an internal passage at a certain height in a transverse direction and having a tilted surface perpendicularly symmetrical to the tilted surface of the partition wall 310 of the first channel on another side such that each fluid flow inside the first channel 300 and the second channel 400 is easily controllable through a tilt.

The microfluidic device may further include: a first separation wall 500 formed while the first channel 300 and the second channel 400 contact with each other; a second separation wall 600 formed while the conduit installation part 200 and the second channel 400 contact with each other; a conduit external channel 700 formed by a spaced apart space between the biomimetic conduit 210 and the conduit installation part 200 when the biomimetic conduit 210 is held; and a conduit holding partition wall 800 installed while extending downwards from the second separation wall 600 such that the second channel 400 and the conduit external channel 700 form a closed space when the biomimetic conduit 210 is held.

The conduit holding partition wall 800 may further include a slit 810 for holding the conduit; a shielding portion 820 for shielding a lower portion of the slit 810; and a fluid guiding partition wall 830 for guiding the fluid introduced from the first channel 300 to the biomimetic conduit 210.

The microfluidic device may further include fluid storage parts 311 and 411 formed on the opposite sides of the tilted surfaces of the partition wall 310 of the first channel and the partition wall 410 of the second channel such that the fluids can be easily introduced thereinto, respectively.

The conduit installation part 200 may hold the biomimetic conduit 210, but the biomimetic conduit 210 may include different cells on an outer side and an inner side, respectively, and the cells may be those different from each other and selected from a group consisting of fibroblasts and epithelial cells.

Another embodiment is a method of culturing a cell using the microfluidic device for mimicking biological tissue, including: holding the biomimetic conduit 210 comprising different cells in a slit 810 of the conduit holding partition wall 800; injecting different culture solutions into the first channel 300 and the second channel 400, respectively; and repeating, for a certain period of time, a process of tilting the frame 100 in one direction such that only a culture solution injected into any one among the first channel 300 and the second channel 400 flows, and then tilting the frame 100 in the opposite direction to the one direction such that only a culture solution in a remaining channel solution among the first channel 300 and second channel 400 flows.

According to an embodiment of the present disclosure, since a channel having a tilted surface is formed in a channel formed in a microfluidic device for mimicking biological tissue, the fluid flow in the channel is easily controllable according to the tilted direction of the device.

In addition, if a fluid storage part capable of easily temporarily storing fluid is further formed on the opposite side of the tilted surface of the partition wall formed in the channel, the velocity of the fluid becomes controllable more precisely.

Further, by easily controlling the fluid flow in different channels supplied to the outer and inner sides of the biomimetic conduit through a tilt formed on a tilted partition wall inside the channel, optimized nutrients can be supplied independently to the cells present in the outer and inner sides of the biomimetic conduit and tissue can be cultured and observed without separating each different cells in the tissue, thus an environment that more closely mimics biological tissue can be provided.

It should be appreciated that the advantageous effects of the present disclosure are not limited to the effects described above, but encompass all effects that can be derived from the configurations of the present disclosure disclosed in the detailed description of the disclosure or the appended claims.

### Brief Description of Drawings

FIG. 1 is a perspective view of the microfluidic device for mimicking biological tissue of the present disclosure.
FIG. 2 is a plan view of the microfluidic device for mimicking biological tissue of the present disclosure.
FIG. 3 is an explanatory diagram for explaining a fluid flow in each channel of the microfluidic device for mimicking biological tissue of the present disclosure.
FIG. 4 is an explanatory diagram for explaining a state in which the microfluidic device for mimicking biological tissue of the present disclosure is tilted in order to control a flow of the channel.
FIG. 5 is a perspective view for explaining a shielding portion for shielding a lower side of the conduit in the microfluidic device for mimicking biological tissue of the present disclosure.
FIGS. 6 and 7 are longitudinal cross-sectional views of concave portions of each embodiment for explaining a shape of a partition wall for controlling a flow of a channel.

### Mode For Invention

Hereinafter, the present disclosure will be described with reference to the accompanying drawings. However, the present disclosure may be embodied in several different forms and is not limited to the embodiments described herein. In order to clearly explain the present disclosure in the drawings, parts irrelevant to the description are omitted, and the same reference numerals will be used to refer to the same or similar elements throughout the specification.

The terms used in the present specification are merely used to describe specific embodiments and are not intended to limit the present disclosure. A singular expression includes a plural expression unless a description to the contrary is specifically pointed out in context. It will be further understood that the terms "comprises," "comprising," "includes" and/or "including," when used herein, specify the presence of stated features, integers, steps, operations, elements, and/or components, but do not preclude the presence or addition of one or more other features, integers, steps, operations, elements, components, and/or groups thereof.

Hereinafter, the exemplary embodiments of the present disclosure will be described in detail with reference to the accompanying drawings.

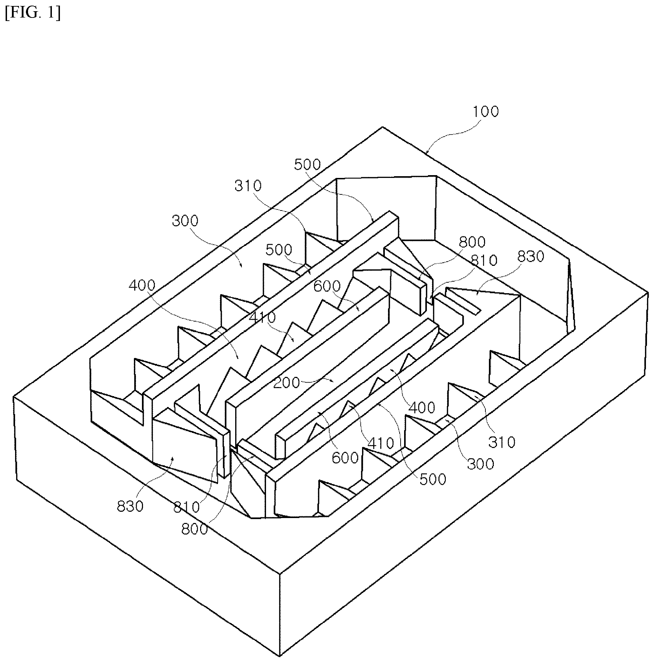

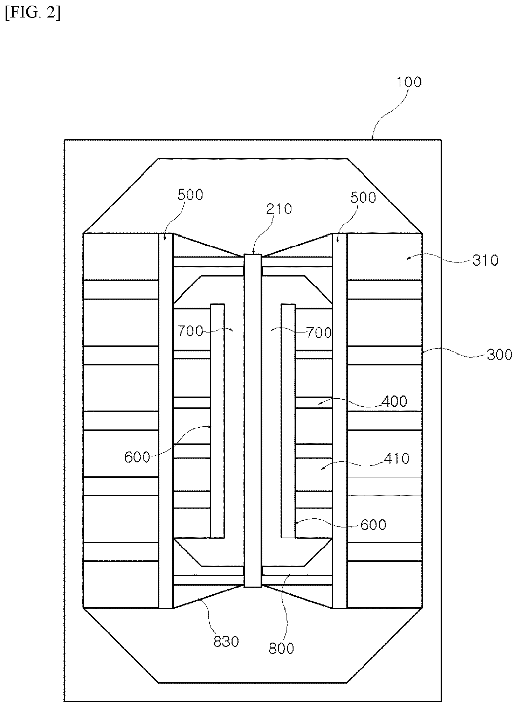

FIG. 1 is a perspective view of the microfluidic device for mimicking biological tissue of the present disclosure, and FIG. 2 is a plan view of the microfluidic device for mimicking biological tissue of the present disclosure. As illustrated in FIGS 1 and 2, the present disclosure is characterized in including a frame 100 and a conduit installation part 200 for holding a biomimetic conduit 210 installed inside the frame 100, and further includes a first channel 300 having at least one or more partition walls 310, which partition an internal passage at a certain height in a transverse direction and allows one side thereof to have a tilted surface.

Since a tilted surface is formed on one side of the partition wall 310 partitioning the internal passage of the first channel 300, it is possible to easily control the speed and flow of the fluid according to a degree and direction of the tilt of the device. That is, when the fluid flowing through the inner passage of the first channel 300 flows in the opposite direction from the one side where the tilted surface is formed, the fluid may freely move along the tilted surface, but conversely, when the fluid flows in the direction of the tilted surface from the opposite surface on which the tilted surface is not formed, the free flow of the fluid is blocked by the partition wall 310.

Preferably, when the fluid flowing through the inner passage of the first channel 300 contains a culture solution required for cells, and the fluid that has passed through the first channel 300 contacts any one among the outer side or inner side of the biomimetic conduit 210 held by the conduit installation part 210, it becomes possible to control the supply of the culture solution to the cells present in the biomimetic conduit 210 contacting the fluid.

The microfluidic device for mimicking biological tissue according to the present disclosure further includes a second channel 400 arranged in parallel with the first channel, and in the second channel 400, at least one or more partition walls 410 partitioning an internal passage at a certain height in a transverse direction and allowing a tilted surface perpendicularly symmetrical to the tilted surface of the partition wall 310 of the first channel to be formed on another side are formed. Through this configuration, directions of each different fluids flowing inside the first channel 300 and the second channel 400 may be controlled opposite to each other.

That is, since the tilted surface of the partition wall 410 formed in the second channel 400 is formed in a direction perpendicularly symmetrical to the tilted surface formed in the partition wall of the first channel 300, when the device is tilted to one side, the fluid inside any channel among the first and second channels 300 and 400 may move, and the movement of the fluid inside the other remaining channel is restricted by the partition wall.

Preferably, there is an advantageous effect in that when the respective fluids flowing through the inner passages of the first and second channels 300 and 400 include culture solutions that are different from each other, and the different fluids that have passed through the respective first and second channels 300 and 400 come into contact with the outer side and the inner side held by the conduit installation part 200, respectively, each different culture solutions may be supplied independently to the different cells present in the outer side and the inner side of the biomimetic conduit 210.

To this end, it is preferable that the first channel 300 and the second channel 400 come into contact with each other to form a first separation wall 500, and the conduit installation part 200 and the second channel 400 come into contact with each other to form a second separation wall 600. In addition, it is preferable to adjust a width of the conduit installation part 200 such that the conduit external channel 700 is formed by a distanced space between the biomimetic conduit 210 and the conduit installation part 200, when the biomimetic conduit 210 is held in the conduit installation part 200. It is preferable to further include a conduit holding partition wall 800 installed while extending downwards from the second separation wall 600 such that the second channel 400 and the conduit external channel 700 form a closed space.

More preferably, as illustrated in FIGS. 1 and 2, forming pairs of the first and second channels 300 and 400; the first and second separation walls 500 and 600; and the conduit external channel 700 to be disposed symmetrically on an upper side and a lower side with respect to the conduit installation part 200 may increase the fluid flow and increase the efficiency of culture.

The biomimetic conduit 210 held by the conduit installation part 200 according to the present disclosure is a biological tissue mimic, and includes an extracellular matrix (ECM) or cells, more preferably, it may be a biological tissue mimic in a form where each different cells are surrounded by an extracellular matrix (ECM) therein. For example, the biomimetic conduit 210 may be a liver tissue in which cells located on the outer side and inner side of the tissue are different from each other, and each of the cells may be selected differently from each other from a group consisting of vascular cells, fibroblasts and epithelial cells.

The biomimetic conduit 210 may be manufactured through 3D printing, and if necessary, may be manufactured by inoculating and culturing cells on a scaffold. In the present disclosure, the biomimetic conduit 210 preferably is the scaffold having a flexible tubular shape in which cells can be cultured therein.

The biomimetic conduit 210 is preferably manufactured such that different cells are cultured and located on the inside and outside of the conduit 210, respectively, in order to mimic biological tissue more similarly.

When the biomimetic conduit 210 is applied to a microfluidic device for simulating biological tissue, as shown in FIGS. 1 and 2, it is possible to independently supply suitable nutrients to each of the cells on the outer side and on the inner side of the biomimetic conduit 210 simply by tilting the device without a separate pump. Through this, metabolism including nutrient supply to cells cultured on the inner side and the outer side of the biomimetic conduit 210 may be achieved through an independent circulatory system.

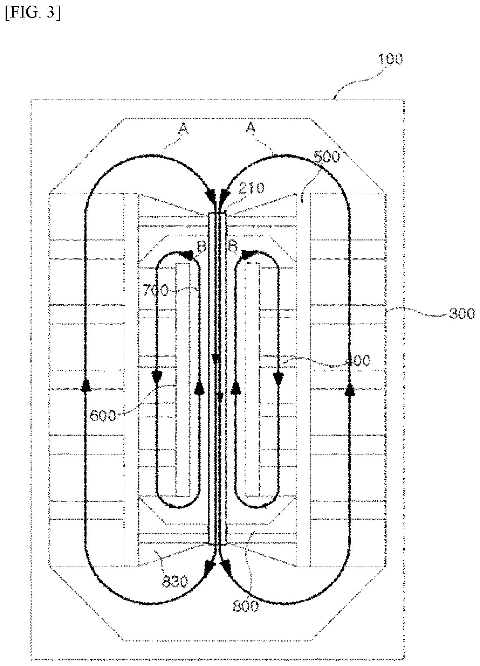

FIG. 3 is an explanatory diagram for explaining a fluid flow in each channel of the microfluidic device for mimicking biological tissue of the present disclosure. The inner side of the biomimetic conduit 210 communicates with the first channel 300 to form a first fluid circulation system A. Through this, cells existing on the inner side of the biomimetic conduit 210 are supplied with a medium required for their culture through the fluid of the first fluid circulation system A.

In addition, the outer side of the biomimetic conduit 210 comes into contact with a second fluid circulation system B formed by mutual communication between the second channel 400 and the conduit external channel 700. Through this, the cells present on the outer side of the biomimetic conduit 210 are supplied with a medium necessary for their culture through the fluid of the second fluid circulation system B.

Meanwhile, it is preferable that the biomimetic conduit 210 is detachable for the convenience of cell culture, and the like. To this end, the device of the present disclosure preferably includes a slit 810 for holding the conduit in the conduit holding partition wall 800; a shielding portion 820 for shielding a lower side of the slit 810; and a fluid guiding partition wall 830 for guiding the fluid introduced from the first channel 300 to the biomimetic conduit 210. By inserting both ends of the biomimetic conduit 210 into the both slits 810, the conduit 210 is fastened to the microfluidic device.

The fluid guiding partition wall 830 is connected to a coupling portion between the first separation wall 500 and the conduit holding partition wall 800 such that the fluid flowing from the first channel 300 to the mimetic conduit 210 (or the same applied in a reverse flow) flows smoothly without a vortex.

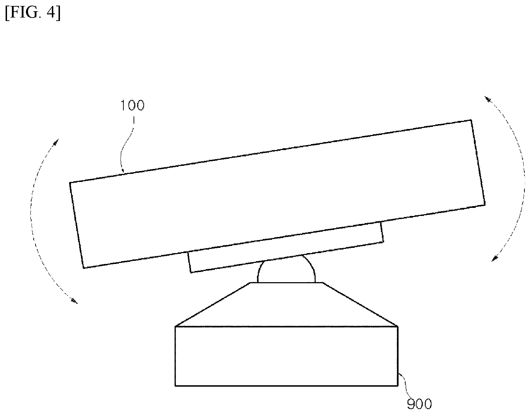

FIG. 4 is an explanatory diagram for explaining a state in which the microfluidic device for mimicking biological tissue of the present disclosure is tilted in order to control a flow of the channel. The present disclosure implements the first fluid circulation system A and the second fluid circulation system B, and according to the requirements of the experimental conditions, being able to select an alternative circulation among the above circulation systems will satisfy more diverse experimental conditions. Accordingly, as described above, according to the present disclosure, partition walls 310 and 410 having tilts are formed in the first channel 300 and the second channel 400. Accordingly, as shown in FIG. 4, when the frame 100 is tilted by a rocker or the like, which is a device that imparts a gradient through tilting, the fluid is shielded by walls opposite to the tilted wall of the partition walls 310 and 410, and the flow is stopped. That is, when tilted to one side, only the first fluid circulation system A is circulated, and when tilted to the other side, only the second fluid circulation system B is circulated. In this configuration, a circulating fluid can be selected by simply tilting, so that more diverse experimental conditions can be easily implemented. In a parallel state without a tilt, both the first fluid circulation system A and the second fluid circulation system B may circulate. For the above configuration, it is preferable that a height of each of the partition walls 310 and 410 is 40 to 80% of a depth of each of the first channel 300 and the second channel 400, respectively.

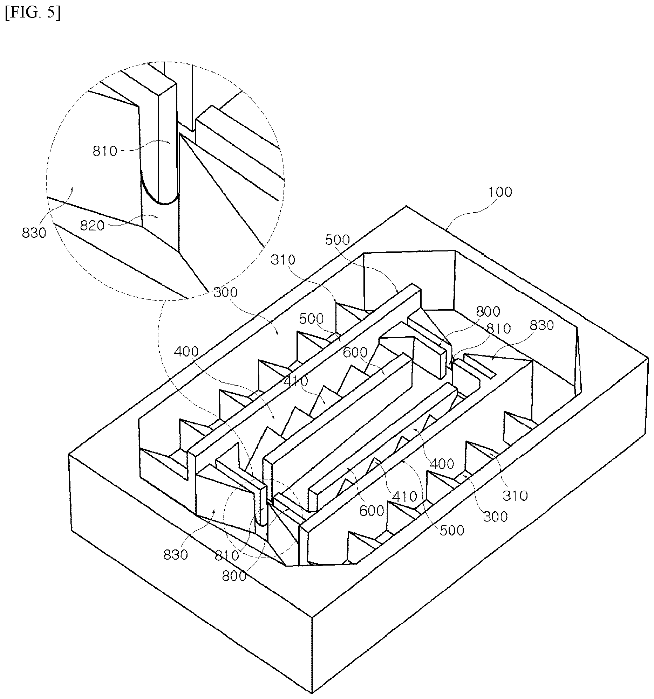

FIG. 5 is a perspective view for explaining the shielding portion for shielding a lower side of the conduit in the microfluidic device for mimicking biological tissue of the present disclosure. The shielding portion 820 for shielding the lower side prevents the fluids flowing toward the fastened biomimetic conduit 210 from the first fluid circulation system A and the second fluid circulation system B, respectively, from mixing with each other. As will be described later, as the present disclosure provides a configuration for shaking the microfluidic device right and left, if there is a risk of an overflow depending on an amount of a fluid, a shielding portion may be provided on an upper side of the biomimetic conduit 210 as well. The shielding portion formed on the upper side may be formed symmetrical with the shielding portion 820, or separate shielding portions may be formed at both ends of the biomimetic conduit 210.

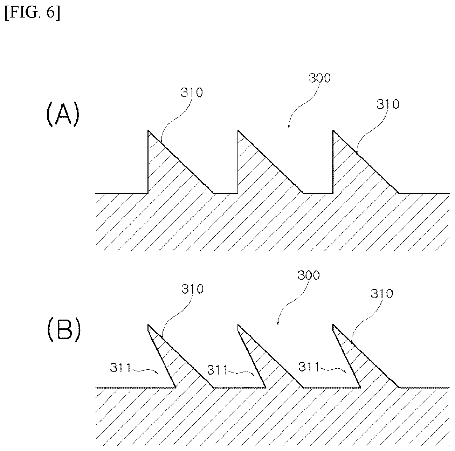

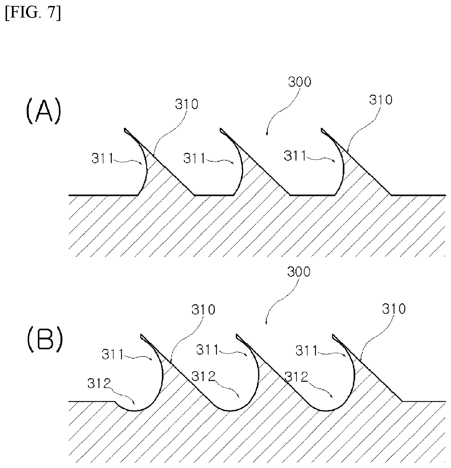

FIGS. 6 and 7 are longitudinal cross-sectional views of concave portions of each embodiment for explaining a shape of the partition wall for controlling a flow of the channel. As shown in (A) of FIG. 6, the opposite side of the tilted surface of the partition wall 310 may have a vertical wall. However, as described above, in order to secure a larger flow rate while preventing an overflow when applying a gradient for selective circulation, the present disclosure provides a structure in which fluid storage parts 311 and 411 are formed on the opposite sides of the tilted surfaces of the partition wall 310 of the first channel and the partition wall 410 of the second channel such that the fluids can be easily introduced thereinto, respectively.

The fluid storage parts 311 and 411 may be provided with an inwardly tilted surface as shown in FIG. 7(A) or may be configured using a curved surface as shown in FIG. 7(B). In addition, as needed, a configuration in which a fluid storage groove 312 is formed on a bottom surface in order to accommodate more fluid is also possible.

In order to mimic an environment where cells on the outer side and inner side of a biological tissue are different from each other, the present disclosure according to the above configuration provides the microfluidic device for mimicking a biological tissue, which can easily control directions of different fluids circulated on the outer and inner sides of the mimetic tissue, respectively, and provides the structure capable of controlling the fluid flow in a specific channel by tilting the frame by forming the partition walls having the tilted surfaces in each channel through which the fluid flows.

Hereinafter, a method of culturing different cells in a biological tissue mimic using the microfluidic device for mimicking a biological tissue according to the present disclosure will be described.

The method of culturing different cells in the biological tissue mimic includes the following operations.

### 1. Manufacturing the biomimetic conduit 210

First, the biomimetic conduit 210 including an extracellular matrix and different cells is manufactured. However, it is not necessarily limited to the conduit formed by the following method.

The biomimetic conduit 210 according to the present disclosure may be composed of two or more layers including an outer layer and an inner layer, and the outer layer and the inner layer may be integrally formed by including neutralized hybrid pregel and CPF-127 gel, respectively. At this time, the neutralized hybrid pregel may be formed of an alginate solution, decellularized extracellular matrix (ECM), DMEM, and cells (typically vascular cells, fibroblasts, epithelial cells, and the like), and the CPF-127 gel may be formed of calcium chloride and F-127.

The biomimetic conduit 210 may be finally manufactured by connecting the inner layer and the outer layer including the gel to a co-axial nozzle, respectively, and applying a pneumatic pressure thereto.

### 2. Holding the mimetic conduit 210

Both ends of the manufactured biomimetic conduit 210 are held by inserting the both ends into both slits 810 of the conduit holding partition wall 800. Through this, the first fluid circulation system A and the second fluid circulation system B are formed independently, and the culture solutions injected into each becomes independently circulated without mixing.

### 3. Injecting culture solutions

Different culture solutions are injected into the first channel 300 and the second channel 400, respectively. The culture solution injected into the first channel 300 may circulate through the first fluid circulation system A formed by the inner side of the biomimetic conduit 210 and the first channel 300. In addition, the culture solution injected into the second channel 400 may circulate through the second fluid circulation system B formed by the second channel 400 and the conduit external channel 700.

### 4. Flowing the culture solution

In a state where the frame 100 is tilted in one direction by a rocker or the like, which is a device that tilts and imparts a gradient, only the culture solution injected into any one among the first and second channels 300 and 400 flows, and the culture solution injected into the remaining channel cannot flow due to the partition wall. When the frame 100 is tilted in the opposite direction to the one direction after a certain period of time has elapsed, only the culture solution in the other channel among the first and second channels 300 and 400 flows. By repeating this series of processes for a certain period of time, it is possible to independently supply different culture solutions to cells at different locations without a separate device such as a pump, just through a gradient by tilting.

The present disclosure may provide nutrients suitable for different cells independently by easily controlling the fluid flows of different channels supplied to the outer and inner sides of the biomimetic conduit 210 using partition walls having tilted surfaces formed inside each channel, accordingly, these cells can be cultured and observed within a single biological tissue mimic without separating different cells in the tissue, thereby providing a more mimicked environment to the biological tissue.

It will be apparent to those skilled in the art that the present disclosure is not limited to the embodiment described above. Thus, the range of the present disclosure should be interpreted based on the following claims, and all technical ideas, including various modifications, additions, substitutions, and the like made without departing from the subject matter of the present disclosure, that fall within the range equivalent to the claims should be understood as belonging to the scope of the present disclosure. In addition, it is noted that some elements in the drawings are provided to more clearly describe the configuration of the present disclosure and are exaggerated or reduced compared to their actual states. In addition, the reference numerals described in the following claims are merely provided to assist understanding and are not intended to limit the shape or the structure of the present disclosure to the accompanying drawings.

## Claims

1. A microfluidic device for mimicking biological tissue, comprising:
a frame 100; and
a conduit installation part 200 for holding a biomimetic conduit 210 installed inside the frame 100,
wherein the microfluidic device comprises a first channel 300 having at least one partition wall 310 partitioning an internal passage at a certain height in a transverse direction and having one side allowing a tilted surface formed therein such that a fluid flow inside the first channel 300 is easily controllable through a tilt.

2. The microfluidic device of claim 1, further comprising:
a second channel 400 arranged in parallel with the first channel,
wherein the second channel 400 has at least one or more partition walls 410 partitioning an internal passage at a certain height in a transverse direction and having a tilted surface perpendicularly symmetrical to the tilted surface of the partition wall 310 of the first channel on another side such that each fluid flow inside the first channel 300 and the second channel 400 is easily controllable through a tilt.

3. The microfluidic device of claim 2, further comprising:
a first separation wall 500 formed while the first channel 300 and the second channel 400 contact with each other;
a second separation wall 600 formed while the conduit installation part 200 and the second channel 400 contact with each other;
a conduit external channel 700 formed by a spaced apart space between the biomimetic conduit 210 and the conduit installation part 200 when the biomimetic conduit 210 is held; and
a conduit holding partition wall 800 installed while extending downwards from the second separation wall 600 such that the second channel 400 and the conduit external channel 700 form a closed space when the biomimetic conduit 210 is held.

4. The microfluidic device of claim 3,
wherein the conduit installation part 200 holds the biomimetic conduit 210, the biomimetic conduit 210 comprises different cells on an outer side and an inner side, respectively, and
the different cells are cells selected from a group consisting of vascular cells, fibroblasts, and epithelial cells and different from each other.

5. A method of culturing a cell using the microfluidic device for mimicking biological tissue of claim 4, comprising:
holding the biomimetic conduit 210 comprising the different cells in a slit 810 of the conduit holding partition wall 800;
injecting different culture solutions into the first channel 300 and the second channel 400, respectively; and
repeating, for a certain period of time, a process of tilting the frame 100 in one direction such that only a culture solution injected into any one among the first channel 300 and the second channel 400 flows, and then tilting the frame 100 in the opposite direction to the one direction such that only a culture solution in a remaining channel among the first channel 300 and second channel 400 flows.
